Europäisches Patentamt

European Patent Office

Office européen des brevets

(11) Publication number: **0 321 709**
A2

## (12) EUROPEAN PATENT APPLICATION

(21) Application number: 88119392.4

(22) Date of filing: 22.11.88

(51) Int. Cl.⁴: **A61K 35/78** , //(A61K35/78, 37:18,33:00,31:765,31:10)

(30) Priority: 23.12.87 US 137081

(43) Date of publication of application:
28.06.89 Bulletin 89/26

(84) Designated Contracting States:
AT BE CH DE ES FR GB GR IT LI LU NL SE

(71) Applicant: Izundegui MacDonnell, Francisco
José
1 de Mayo 222 Colonia Garcia
MX-86040 Villahermosa Tabasco(MX)

(72) Inventor: Izundegui MacDonnell, Francisco
José
1 de Mayo 222 Colonia Garcia
MX-86040 Villahermosa Tabasco(MX)

(74) Representative: Dorner, Jörg, Dr.-Ing.
Dorner & Hufnagel Patentanwälte
Ortnitstrasse 20
D-8000 München 81(DE)

(54) **Fast healing and binding chemical compound or curative agent for healing wounded live animal and human soft tissues inflicted by cutting edges or separating forces.**

㊄ There is disclosed here a chemical compound or curative agent in which formula enters a number of substances among them a new one, a resin from the cortex of the plant Mimosa tuitenuiflora leguminosae Poir with a perfect -compatible and stable nature in the form of a viscous liquid. Said Chemical compound acts as a curative agent when applied on wounds or cutts of live animal and human soft tissues causing them to become healed or bound in a short period of time that takes from 2 minutes to 30 minutes ( depending on the nature of the tissue involved ) without failure and prompt restoration of the functional activity of the tissues and not leaving visible scar and not allowing kelloid degeneration. Said chemical compound also -acts as a carrier for binding single isolated live cells inside all live animal and human soft tissues taken from either said beens or from cell cultures. Also can be used to transplant viable animal or human eye for restoration of vision.

# Fast healing and binding chemical compound or curative agent for healing wounded live animal and human soft tissues inflicted by cutting edges or separating forces.

The invention relates to a chemical compound to be used in the field of the Medical and Surgical Arts, specifically to an original chemical compound, which has prouved it's effectiveness and lacking harmfull side effects during continious use and medical experimentation upon been smeared on the raw opposite surfaces of severed or wounded live animal and -human soft tissues, such as: skin, fascia, fat, muscle, tendon, ligament, all glandular tissue, all organ's tissue, blood vessels, all membranes, mucosas, nerves and spinal cord which causes on them, while been kept in full contact and firmly to become healed and bound together in a short period of time that takes from 2 minutes to 30 minutes depending on the nature of the tissue so treated , providing that the chemical compound is not removed from while it's action is taking place. Therefore can be concidered into the field of drugs.

Background and discussion of prior Art.

The Medical and Surgical profesions has always been confronted with multiple problems that aroused from prolonged, incomplete, disrupted, enlarged, degenerated, failures of healing, horrible looking scar formation or kelloid degenearation, retarded healing, pressure causing malfunction or pain by the presence of exagerated scar bulky formation and many other situations such as paralysis or lack of sensibility, grafting failure, etc, that happens to occur when dealing with wounds inflicted on soft tissues by means of cutting edges or separating forces. No practical or effective means other than the usual approximating devices as sutures of different kinds, bandages, nnedles, staples, hooks, have been used before to the advent of my invention that will cause to heal and bind in a fast, rapid, complete and firm modality without failure in all cases and also without leaving degenerated or even visible scars, preventing kelloid degeneration. Functional recovery takes place promptly and usually full functioning is restored in a short period of time even for nerves that has been severed. The natural healing process that usually takes place after wounding of -soft tissues is done through fibrin from blood meshlike deposition plus other elements as plateletes and posteriorly fibroblastic proliferation and collagen deposition that upon dehaydration becomes organiced and hardened forming the well known scar tissue. This one of different histological nature as the tissue involved. The time elapsed for this natural healing proces to occur goes for five to ten days and some times never heals at all. Many pathological conditions excerts it's negative influence on -wound healing. Wherefore there was not until now any existing mean to produce by artificial procedure to secure a fast, firm, non obstructing, functional, non differing from the wounded tissue histological structure and -that will not leave visible scar or cause to avoid the appearance of kelloid degeneration and functional failure.

## Objects and advantages

Most Medical and Surgical profesionists world wide will wellcome for their patients convinience and themselves the advent of giving to them an invention as this chemical compound or curative agent now been applied for pa-tentability, that has the propriety then to cause fast healing and binding of live animal and human soft tissues wounded by cutting edges or separating forces without failure, diminishing suffering and shortening convalescence. Accordingly I claim the following as my objects and advantages of the invention and it's use: To provide a chemical novel compound or curative agent with an unusual capacity for healing to the Veterinarian and Medical profesions and related arts for easily, reliably, neatly, with rapid functional recovery, without visible scar formation, with complete anatomical and histological self nature integration of the cellular elements of the tissues, without failures, in a shost period of time that goes from 2 minutes to 30 minutes for all wounded live animal tissues such as: skin, fascia, muscle, tendosns, ligaments, all glandular tissue, oll organ's tissues, blood vessels, mucosas, nerves, membranes, spinal cord tissues and pariostium which has been wounded by cutting edges or separating forces. In adition I claim the following advantages to the Medical, Surgical and Veterinarian Arts or profesions to favor them with a safe. reliable, nonpareil, non noxious, easy to use and that reduces to a minimun or none at all, the usual wound healing complications saving time, suffering, expenditures, legal affairs and achieving sound sudden recovery.

Description of the invention

Stable chemical compound, viscous, fluid transparent, amber coloured, Ph 7.4 composed with the following elements:

1. A solution of mineral salts and aminoacids.
2. A synthetic polymer of variable density 4000 to 8000.
3. A natural polymer ( resin ).
4. An organic solvent.

Disclousure and coposition of each of the above elements:

1. A solution of mineral salts and aminoacids made with the following described by name and amounts used:

| Aminoacids: | |
|---|---|
| L- Leucine | 500 mgs |
| L- Phenyilalanine | 500 mgs |
| L- Methionine | 500 mgs |
| L- Lysine hydrochloride | 500 mgs |
| L- Isoleucine | 400 mgs |
| L- Valine | 400 mgs |
| L- Histidine Hydrochloride | 400 mgs |
| L- Threonine | 400 mgs |
| L- Ttyptophan | 150 mgs |
| L- Alanine | 1.5 Gms |
| L- Arginine hydrochloride | 800 mgs |
| L- Proline | 300 mgs |
| L- Tyrosine | 30 mgs |
| Salts: | |
| Sodium acetate | 600 mgs |
| Dibasic potassium phosphate | 500 mgs |
| Sodium chloride | 150 mgs |
| Magnesium chloride | 100 mgs |

Inyectable destilled water amount sufficient to make 1000 Ml.

2- A synthetic polymer of variable density 4000to 8000:
Melted polyethyleneglycol     650 Mls

3- A natural Resin from Mimosa Tuitenuiflora Leguminosae Poir plant 2 Gms

4- An organic solvent Dimethyl sulphoxide     80 Mls

Manufacturing procedure:

Dissolve the aminoacids and salts in destilled water in the amounts given in 1. sufficient to make 1000 Mls.

Melt 4000,6000 or 8000 polyethylenglycol by slowly heating under controled temperature at no more than 60 degrees Centigrades to make a volume of 650 Mls melted material.

While still been warm mixe both 1 and 2 by stirring.

Add 3 and 4 mixed, acting the dimethyl sulphoxide as solvent for the Mimosa Tuitenuiflora Resin.

Bring into Ph 7.4 by adding either NaOH or HCL 0.1 M solution as much as required.

Sterilize by autoclave and keep in a dealed glass container.

Ready to be used.

Procedure to extract the Resin from the cortex of 5 year old Mimosa TuiTenuiflora Leguminosa Plant:

Take 20 Grams of dried cortex from Mimosa TuiTenuiflora Leguminosae plant grind it to a fine grain and extract with 100 Mls of the organic solvent Dimethyl sulphoxide.

Filter and keep in a closed glass container for posterior use. Amount of solvent recovered 80 Mls with 2 Gms Resin dissolved.

Conclusion ans scope of the invantion

Thus the reader will see that while my above description contains many specificities, this should not be concidered as limitations on the scope of the invantion. Many other variations are possible. For example skilled of the Art persons will be able to change the nature and amount of the compounents. They also will be able to change the density of the synthetic polymer but not the nature of the natural Resin derived from Mimosa tuiTenuiflora Leguminosae Plant. An for the methods for using the chemical compound or curative agent many other variations or applications can be performed such as injectingthe invention through tissues to reach deeper separated structures and as a vehicle for carrying isolated live cells inside any soft tissues of live animals or humans and also grafting free live pieces of tissues separated appart by means of cutting edges( knifes) taken from the same animal or human body or from tissue cultures. Accordingly the scope of the invantion should not be determined by the descriptions and examples given but by the appended claims and their legal equivalents.

## Claims

1- A chemical compound comprising: A- a solution composed by water, mineral salts and aminoacids. B- a synthetic polymer, polyethylenglycol of variable density ( 4000 to 8000 ). C- a natural polymer, a resin from the cortex of the Plant mimosa Tuitenuiflora Leguminosae Poir. D- an organic solvent, dimethyl sulphoxide.

2-The chemical compound of claim 1, acting as a curative agent that causes fast healing and binding on the wounds of live animal and human soft tissues inflicted upon them by the action of cutting edges or disruptive forces.

3- The proprietary action of the chemical compound of claim 1, acting as a curative agent as said in claim 2 in a short period of time that takes from 2 minutes to 30 minutes depending on the nature of the said soft tissues.

4- The proprietary action of the chemical compound of claim 1, acting as a curative agent that causes healing and binding of the wounded soft tissues of live animals and humans inflicted on them by the action of cutting edges or disrupting forces , those soft tissues said to be: skin, mucosas, fascia, fat, muscle, tendom, ligament, periostium, blood vessels, nerves, spinal cord tissues, all tissues of glands, all tissues of organs in a --shoert period of time that takes from 2 minutes to 30 minutes, depending on the nature of the tissue involved.

5- The method of generating healing and binding curative action by smearing or applying variable amounts of the chemical compound of claim1, on the surfaces of the exposed cells of live animal and human soft tissues woundes or separeted by the action of cutting edges or disrupting forces as said in claim 4and not leaving visible scars, mainly in the skin.

6- The method of generating healing and binding curative action by injecting variable amounts of the chemical compound of claim 1, inside living animal and human soft tissues separated by the action of disrupting mechanical forces.

7- The method of using the chemical compound of claim 1 as a carrier for isolated live animal or human cells to be bound into all soft tissues of live animals and humans.

8- The method of using the chemical compound of claim 1, as a mean for isolated groups of tissues cells or single cells from either live animal or human soft tissues or from tissue cultures to become bound with the tissue cells of said animals and humans.

9- The method of using the chemical compound of claim 1 as a mean for se for seveared nerves and spinal cord to become healed and bound in a fast and firm manner, with prompt recovery of neurotransmission of motor and sensitive impulses.

10-The method of using the chemical compound of claim 1 as a mean for the eye taken from other live animal or human or from the said animals or humans still viable short after dead to be transplanted and become healed and -bound to the animal or human receptor respectibly for vision recovery.